# EUROPÄISCHE PATENTSCHRIFT

(11) **EP 3 299 036 B1**
(45) Veröffentlichungstag und Bekanntmachung des Hinweises auf die Patenterteilung: **12.06.2019**
(21) Anmeldenummer: 16190798.5
(22) Anmeldetag: 27.09.2016
(51) Int. Cl.: A61L 2/28, A61C 19/00

(54) **VALIDIERUNGSSET SOWIE VERFAHREN ZUM ÜBERPRÜFEN DER REINIGUNGSLEISTUNG EINES REINIGUNGSAPPARATS**
VALIDATION SET AND METHOD FOR TESTING THE CLEANING PERFORMANCE OF A CLEANING DEVICE
ENSEMBLE DE VALIDATION ET PROCÉDÉ DE CONTRôLE DU POUVOIR NETTOYANT D'UN APPAREIL DE NETTOYAGE

(43) Veröffentlichungstag der Anmeldung: 28.03.2018
(73) Patentinhaber: XYLEM Analytics Germany GmbH, 82362 Weilheim (DE)
(72) Erfinder: Streller, Robert, 92339 Beilngries (DE); Beyer, Klaus, 85055 Ingolstadt (DE)
(74) Vertreter: Bergmeier, Werner

(56) Entgegenhaltungen:
- EP-A1- 1 952 782
- EP-A1- 2 514 386
- WO-A1-96/04936
- WO-A1-2014/016011
- WO-A2-2010/106161
- DE-B3-102004 060 289
- US-A- 5 971 757
- US-A1- 2002 119 416

## Beschreibung

Die vorliegende Erfindung entstammt dem technischen Gebiet der Reinigung von Dentalinstrumenten und deren Überprüfung.

EP2514386 offenbart mehrere Adapter für Dentalinstrumente und deren Befestigung an einer Deckelplatte eines Reinigungsapparats, wobei der Adapter durch integrierte Sensoren das Reinigungsverfahren und die darin erreichten Temperaturen festhält. Die Erfindung wird durch die unabhängigen Ansprüche definiert. Gattungsgemäße Reinigungsapparate sind seit langem bekannt und dienen der Reinigung von zahnärztlichen Handgeräten (wobei im Rahmen der vorliegenden Erfindung ein Reinigungsvorgang auch einen Desinfizier- und/oder Sterilisierungsabschnitt umfassen kann). Unter zahnärztlichen Handgeräten werden vorliegend all diejenigen Gerätschaften verstanden, die ein Zahnarzt beim Behandeln eines Patienten einsetzt und die einen, meist länglichen, Griff zum Greifen und Führen der Gerätschaft sowie einen sich an den Griff anschließenden Funktionsabschnitt aufweisen. Bei dem Funktionsabschnitt handelt es sich beispielsweise um eine Aufnahme für ein rotierendes Werkzeug (z. B. einen Zahnbohrer oder einen Zahnfräser) oder eine Saugdüse, über die während der Zahnbehandlung Speichel und andere Flüssigkeiten aus dem Mundraum des Patienten abgeführt werden können.

In jedem Fall ist es nötig, derartige Handgeräte nach Beendigung der Behandlung eines Patienten vollständig zu reinigen, um eine Übertragung von Mikroorganismen auf einen Patienten zu verhindern, der im Anschluss mit denselben Handgeräten behandelt wird.

Die Handgeräte werden hierzu in die Reinigungskammer eines entsprechenden Reinigungsapparats gegeben und dort mit jeweils einem Aufnahmestutzen verbunden. Anschließend wird die Reinigungskammer mit Hilfe eines Deckels luftdicht verschlossen. Im nachfolgenden Reinigungsschritt (der generell und auch im Rahmen der vorliegenden Erfindung einen Desinfektions- und/oder Sterilisierungsschritt umfassen kann) werden über den Aufnahmestutzen eine oder mehrere Fluide (z. B. reines oder mit Zusatzstoffen versetztes, vorzugsweise erhitztes, Wasser oder Dampf) in und durch das Handgerät geleitet, um Verschmutzungen im Inneren des Handgeräts zu entfernen. Ebenso erfolgt meist eine Reinigung der Handgeräte von außen, in dem deren Außenseite mit einem oder mehreren Fluiden behandelt wird.

Im Ergebnis erhält man nach dem Reinigungsvorgang gereinigte zahnärztliche Handgeräte, die erneut für die Behandlung eines Patienten eingesetzt werden können.

In regelmäßigen Zeitabschnitten ist es nun nötig, die Funktionsweise, d. h. die Reinigungsleistung des Reinigungsapparats, zu überprüfen, um sicherzustellen, dass die mit dessen Hilfe gereinigten zahnärztlichen Handgeräte auch tatsächlich gereinigt (und je nach Art des Reinigungsapparats vorschriftsmäßig desinfiziert bzw. sterilisiert) werden.

Bekannt sind in diesem Zusammenhang separate Deckel zum Verschließen der Reinigungskammer, an denen Metallkörper befestigt sind, die die eigentlich zu reinigenden zahnärztlichen Handgeräte simulieren. Diese werden kontrolliert verschmutzt (z. B. mit künstlichem Blut oder Schafsblut) und anschließend in dem Reinigungsapparat einer Reinigung unterzogen. Im Anschluss daran wird die Restverschmutzung der Metallkörper überprüft und daraus auf die Reinigungsleistung des Reinigungsapparats geschlossen.

Der Nachteil an diesem Verfahren liegt vor allem in der Tatsache, dass der Deckel des Reinigungsapparats, der normalerweise Verwendung findet, während der Validierung durch einen anderen Deckel ersetzt wird. Das Reinigungsergebnis, das während der Validierung erhalten wird, entspricht damit nicht unbedingt der tatsächlichen Reinigungsleistung des Reinigungsapparats.

Aufgabe der vorliegenden Erfindung ist es daher, ein Validierungsset zum Überprüfen der Reinigungsleistung eines zum Reinigen von zahnärztlichen Handgeräten ausgelegten Reinigungsapparats, dessen Verwendung sowie ein Verfahren zum Überprüfen der Reinigungsleistung entsprechender Reinigungsapparate vorzuschlagen.

Die Aufgabe wird gelöst durch die Gegenständer der unabhängigen Patentansprüche.

Das erfindungsgemäße Validierungsset umfasst nun zumindest einen Adapter mit einem ersten und einem zweiten Anschlussbereich. Während der erste Anschluss ausgelegt ist, mit einem der Aufnahmestutzen des Reinigungsapparats gekoppelt zu werden, ist der zweite Anschlussbereich ausgelegt, mit einem Prüfkörper gekoppelt zu werden, wobei auch der Prüfkörper ebenfalls Teil des Validierungssets ist. Der Adapter ist vorzugsweise zumindest größtenteils aus Metall gefertigt, wobei der erste und der zweite Anschlussbereich vorzugsweise auf gegenüberliegenden Stirnseiten des Adapters platziert sind. Der erste Anschlussbereich kann beispielsweise als Vertiefung ausgebildet sein, in die der Aufnahmestutzen des Reinigungsapparats nach der Kopplung mit dem Adapter ragt. Prinzipiell ist es von Vorteil, wenn der erste Anschlussbereich Abschnitte eines Schnappverschlusses umfasst, so dass der Adapter genauso auf einen Aufnahmestutzen des Reinigungsapparats gesteckt werden kann wie ein herkömmliches zahnärztliches Handgerät.

Der zweite Anschlussbereich ist schließlich derart ausgebildet, dass der Prüfkörper des Validierungssets mit dem Adapter in dem genannten Bereich gekoppelt werden kann. Auch der zweite Anschlussbereich kann als Vertiefung vorliegen, in die ein korrespondierender Abschnitt des Prüfkörpers gesteckt werden kann (selbstverständlich kann auch der Prüfkörper eine Vertiefung aufweisen zur Aufnahme eines Abschnitts des zweiten Anschlussbereichs des Adapters).

Durch die Gestaltung des ersten und zweiten Anschlussbereichs des Adapters ist es schließlich möglich, den Prüfkörper mit dem Adapter und über den Adapter mit dem Reinigungsapparat, d. h. mit einem seiner Aufnahmestutzen, zu verbinden. Der Prüfkörper kann vor der Durchführung eines Reinigungsvorgangs kontrolliert verschmutzt und anschließend mit dem Reinigungsapparat gekoppelt werden. Der Prüfkörper dient somit gemeinsam mit dem Adapter als Ersatz eines zahnärztlichen Handgeräts. Nach Durchführung des Reinigungsvorgangs kann schließlich der Prüfkörper hinsichtlich noch vorhandener Verunreinigungen untersucht und damit die Reinigungsleistung des Reinigungsapparats bewertet werden.

Der wesentliche Vorteil liegt nun darin, dass während der Validierung ausschließlich die Bauteile des Reinigungsapparats zum Einsatz kommen, die auch während der routinemäßigen Reinigung zahnärztlicher Handstücke Verwendung finden. Fehlfunktionen des Reinigungsapparats können hierdurch wesentlich zuverlässiger detektiert werden als im Stand der Technik, bei der der Originaldeckel während der Validierung durch einen separaten Deckel ersetzt wird.

Insbesondere ist es von Vorteil, wenn das Validierungsset mehrere Adapter umfasst, wobei zumindest die ersten Anschlussbereiche zweier Adapter unterschiedlich ausgebildet sind, um die jeweiligen Adapter mit unterschiedlich ausgebildeten Aufnahmestutzen koppeln zu können. Hintergrund ist die Tatsache, dass zahnärztliche Handgeräte von unterschiedlichen Herstellern angeboten werden und deren Anschlussbereiche teilweise voneinander abweichen. Folglich sind auch die Aufnahmestutzen der sich am Markt befindlichen Reinigungsapparate nicht alle gleich ausgebildet. Vielmehr variiert die Geometrie der Aufnahmestutzen in Abhängigkeit der zahnärztlichen Handgeräte, die der jeweilige Zahnarzt einsetzt. Umfasst das Validierungsset nun mehrere Adapter mit unterschiedlich ausgebildeten ersten Anschlussbereichen, so wird die Zahl an Aufnahmestutzen, mit denen zumindest einer der Adapter koppelbar ist, erhöht.

Vorteilhaft ist es, wenn die zweiten Anschlussbereiche aller Adapter gleichartig ausgebildet sind. Während die ersten Anschlussbereiche variieren sollten, um die Adapter mit unterschiedlichen Aufnahmestutzen koppeln zu können, dient der zweite Anschlussbereich dem Koppeln des Prüfkörpers mit dem Adapter. Der Adapter dient also als Zwischenstück zwischen einem Aufnahmestutzen des Reinigungsapparats und dem Prüfkörper und soll eine Verbindung des Prüfkörpers mit unterschiedlichen Aufnahmestutzen ermöglichen. Wenn alle Adapter mit dem Prüfkörper koppelbar sind, muss die die Validierung des Reinigungsapparats durchführende Person lediglich den Adapter mit dem passenden ersten Anschlussbereich wählen. Da alle Adapter denselben zweiten Anschlussbereich aufweisen, kann der Prüfkörper schließlich auch mit jedem Adapter gekoppelt werden.

Vorteilhaft ist es zudem, wenn das Validierungsset mehrere Adaptersätze umfasst. Jeder Adaptersatz umfasst vorzugsweise mehrere gleichartig ausgebildete Adapter. Zudem sollten sich die ersten Anschlussbereiche der Adapter eines Adaptersatzes von den ersten Anschlussbereichen der Adapter des oder der weiteren Adaptersätze unterscheiden. Schließlich ist es von Vorteil, wenn alle zweiten Anschlussbereiche aller Adapter gleich ausgebildet sind. Soll nun die Reinigungsleistung eines Reinigungsapparats überprüft werden, so wird der Adaptersatz mit den Adaptern ausgewählt, deren erster Anschlussbereich mit den Aufnahmestutzen des Reinigungsapparats koppelbar ist. Diese Adapter werden schließlich (vorzugsweise nach vorangegangener kontrollierter Verschmutzung) jeweils mit einem Aufnahmestutzen und jeweils mit einem Prüfkörper verbunden.

Vorzugsweise umfasst das Validierungsset drei gleichartige Prüfkörper. Ebenso sollte jeder Adaptersatz drei gleichartige Adapter umfassen. Dies stellt sicher, dass während des der Validierung des Reinigungsapparats dienenden Reinigungsvorgangs drei Prüfkörper über jeweils einen Adapter mit jeweils einem Aufnahmestutzen verbunden sein können. Die Auswertung der Reinigungsleistung kann in diesem Fall auch mit statistischen Werten (z. B. Konfidenzintervallen) ergänzt werden.

Ebenso bringt es Vorteile mit sich, wenn der Prüfkörper einen den Prüfkörper in Richtung dessen Längsachse durchziehenden Kanal aufweist. Wird nun vom Reinigungsapparat während des Reinigungsvorgangs ein Fluid (z. B. heißer Wasserdampf) abgegeben, so kann das Fluid durch den Prüfkörper strömen und diesen auch von innen reinigen. Wurde der Prüfkörper vor dem Einsetzen in den Reinigungsapparat kontrolliert verschmutzt, so kann aus der nach dem Reinigungsvorgang vorhandenen Restschmutzmenge die Reinigungsleistung abgeleitet werden. Im Übrigen sei an dieser Stelle darauf hingewiesen, dass unter dem Begriff "verschmutzen" im Rahmen der Erfindung ein Vorgang zu verstehen ist, bei dem der Prüfkörper an ausgewählten Stellen (z. B. im Bereich seiner nach außen weisenden Oberfläche und/oder im Bereich des genannten Kanals) mit einer Flüssigkeit benetzt wird, die definierte chemische und/oder biologische Eigenschaften aufweist. Beispielsweise könnte die Flüssigkeit mit einem oder mehreren Mikroorganismen versehen sein, die auch bei Patienten im Mundraum anzutreffen sind.

Vorteilhaft ist es, wenn der oder die Adapter jeweils einen den entsprechenden Adapter in Richtung dessen Längsachse durchziehenden Kanal aufweisen. Wird nun vom Reinigungsapparat ein Fluid über den Reinigungsstutzen abgegeben, so kann dieses durch den Adapter und schließlich auch durch den Prüfkörper strömen, nachdem der Adapter mit einem Aufnahmestutzen des Reinigungsapparats gekoppelt wurde. Adapter und Prüfkörper simulieren in diesem Fall gemeinsam ein zahnärztliches Handstück. Vorzugsweise hat der Kanal einen Durchmesser zwischen 2 mm und 6 mm. Zudem ist es von Vorteil, wenn das Validierungsset eine Vielzahl von (Kunststoff-)Schläuchen umfasst, die in den Kanal des Prüfkörpers und des Adapters gesteckt werden können und die vorzugsweise eine Länge aufweisen, die größer ist als die gemeinsame Länge der Kanäle von Adapter und Prüfkörper. Die Verwendung eines Schlauches hat schließlich den Vorteil, dass vor Durchführung eines Reinigungsvorgangs nicht der Prüfkörper als solcher von innen verschmutzt werden muss. Vielmehr kann der Schlauch von innen verschmutzt und anschließend in das durch Prüfkörper und Adapter gebildete Bauteil geschoben werden.

In diesem Zusammenhang ist es schließlich ebenso von Vorteil, wenn der zweite Anschlussbereich einen Anschluss aufweist, über den der Schlauch fixiert werden kann (der Schlauch besitzt vorzugsweise eine Länge zwischen 10 cm und 15 cm, insbesondere zwischen 11 cm und 13 cm und kann im Übrigen der Norm ISO 15883 entsprechen).

Beispielsweise könnte der Anschluss als Luer-Lock-Anschluss oder IQS-Anschluss ausgebildet sein.

Der zweite Anschlussbereich dient vorzugsweise neben der Fixierung des Prüfkörpers auch der Fixierung eines Schlauchs. Alternativ kann auch der Schlauch allein als Prüfkörper dienen, wenn auf einen zusätzlichen Prüfkörper verzichtet wird.

Besondere Vorteile bringt es mit sich, wenn der erste Anschlussbereich der jeweiligen Adapter zumindest ein Rastelement umfasst, mit dessen Hilfe er mit einem Aufnahmestutzen des Reinigungsapparats durch Bildung einer Schnappverbindung koppelbar ist. Adapter und Aufnahmestutzen können in diesem Fall werkzeuglos gekoppelt und wieder entkoppelt werden. Ebenso wäre es denkbar, dass der Adapter im Bereich seines zweiten Anschlussbereichs ein Innen- oder Außengewinde aufweist, welches mit einem Außen- bzw. Innengewinde im Bereich des Anschlusses des Prüfkörpers verschraubbar ist.

Ferner ist es von Vorteil, wenn die Adapter in ihrem ersten und/oder zweiten Anschlussbereich eine Dichtung aufweisen, um die Verbindung zwischen Adapter und Aufnahmestutzen bzw. zwischen Adapter und Prüfkörper luftdicht gestalten zu können.

Auch ist es äußerst vorteilhaft, wenn das Validierungsset zumindest zwei unterschiedlich ausgebildete Prüfkörper umfasst, mit deren Hilfe während einer Überprüfung der Reinigungsleistung des Reinigungsapparats unterschiedliche Kenngrößen detektierbar sind. Beispielsweise wäre es denkbar, dass ein erster Prüfkörper entsprechend der bisherigen Beschreibung ausgebildet ist und der Überprüfung der vom Reinigungsapparat durchgeführten Entfernung einer definierten Verschmutzung ausgebildet ist. Das Validierungsset umfasst vorzugsweise drei derartige Prüfkörper. Darüber hinaus sollte zumindest ein weiterer Prüfkörper Teil des Validierungssets sein, mit dessen Hilfe Temperatur- und/oder Druckwerte während des Reinigungsvorgangs überprüfbar sind. Beispielsweise wäre es denkbar, dass ein derartiger Prüfkörper ebenfalls einen innenliegenden Kanal aufweist, dessen Durchmesser vorzugsweise 3 mm bis 7 mm aufweist. Durch diesen Kanal kann beispielsweise vor dem Reinigungsvorgang eine Temperatursonde eingesteckt werden, die während des Reinigungsvorgangs den Temperaturverlauf im Inneren des Prüfkörpers überwacht bzw. detektiert.

Vorzugsweise sind alle zweiten Anschlussbereiche ausgebildet, mit den Anschlüssen aller Prüfkörper gekoppelt werden zu können. Besondere Vorteile bringt es in diesem Zusammenhang mit sich, wenn die Anschlüsse aller Prüfkörper, über die sie mit dem genannten zweiten Anschlussbereich eines Adapters koppelbar sind, identisch ausgebildet sind. Mit anderen Worten ist es also von Vorteil, wenn alle Prüfkörper mit allen Adaptern koppelbar sind.

Des Weiteren ist es vorteilhaft, wenn das Validierungsset einen oder mehrere Temperatursensoren und/oder einen oder mehrere Drucksensoren umfasst. Die Sensoren können vor dem Verschließen der Reinigungskammer des Reinigungsapparats in diese eingebracht werden. Während des Reinigungsvorgangs können schließlich Temperatur- und/oder Druckdaten aufgezeichnet und bei der Bewertung der Reinigungsleistung des Reinigungsapparats berücksichtigt werden. Vorzugsweise weisen der oder die Temperatursensoren und/oder der beziehungsweise die Drucksensoren einen Datenspeicher auf, der ausgebildet ist, vom jeweiligen Sensor gelieferte Messwerte für eine spätere Auswertung zu speichern. Die Messwerte werden also während des Reinigungsvorgangs innerhalb der Reinigungskammer detektiert und durch den entsprechenden Sensor selbst gespeichert und können nach Abschluss des Reinigungsvorgangs aus dem Datenspeicher ausgelesen und anschließend im Hinblick auf die Reinigungsleistung des Reinigungsapparats ausgewertet werden.

Vorteile bringt es zudem mit sich, wenn das Validierungsset wenigstens ein Halteelement, vorzugsweise in Form einer Halteklammer, umfasst. Das Halteelement besitzt wiederum eine oder mehrere Aufnahmen zum Halten von Temperatur- und/oder Drucksensoren. Bei den Aufnahmen kann es sich um Öffnungen handeln, durch die jeweils ein Sensor geschoben werden kann. Nach der Ausrichtung des jeweiligen Sensors innerhalb der Öffnung kann dieser schließlich fixiert werden, wobei das Halteelement hierfür vorzugsweise einen oder mehrere Bolzen, Schrauben oder Gewinde zur Aufnahme von Schrauben aufweist, über die der jeweilige Sensor relativ zum Haltelement fixierbar ist. Ferner sollte das Halteelement einen Halteabschnitt aufweisen, über den es mit dem Reinigungsapparat verbindbar ist. Beispielsweise wäre es denkbar, dass der Halteabschnitt durch einen C-förmigen Abschnitt des Halteelements gebildet wird, über den es mit einem zylindrischen Abschnitt des Reinigungsapparats anbringbar ist (entsprechende Abschnitte weisen die am Markt bekannten Reinigungsapparate in der Regel in Form eines in die Reinigungskammer ragenden Fortsatzes auf, so dass hier keine Modifikationen nötig sind).

Vorzugsweise ist der Halteabschnitt federnd ausgebildet, so dass das Halteelement über den zylindrischen Abschnitt in dessen Radialrichtung geschoben werden kann und in der gewünschten Endstellung einschnappt.

Des Weiteren wird ein Verfahren zum Überprüfen der Reinigungsleistung eines zum Reinigen von zahnärztlichen Handgeräten ausgelegten Reinigungsapparats, wie er oben bereits beschrieben wurde, vorgeschlagen. Das Verfahren umfasst zumindest die folgenden Schritte:
Zunächst wird vor der Überprüfung der Reinigungsleistung zumindest einer der Aufnahmestutzen des zu überprüfenden Reinigungsapparats mit einem Adapter und der Adapter mit einem Prüfkörper gekoppelt, wobei der Prüfkörper zuvor oder anschließend kontrolliert verschmutzt wird. Anschließend wird die Reinigungskammer mit Hilfe ihres Deckels verschlossen. Nachfolgend wird ein Reinigungsvorgang durchgeführt, wie er auch beim Reinigen von zahnärztlichen Handgeräten durchgeführt wird.

Die Reinigungsleistung wird schließlich unter Berücksichtigung von während des Reinigungsvorgangs aufgezeichneter physikalischer Daten und/oder unter Berücksichtigung des Reinigungserfolgs des vor Durchführung des Reinigungsvorgangs kontrolliert verschmutzten Prüfkörpers bewertet. Beispielsweise wäre es denkbar, die folgenden Parameter bei der Bewertung zu berücksichtigen:
- Menge der nach dem Reinigungsvorgang noch am Prüfkörper vorhandenen Verunreinigungen (z. B. als Absolutwert oder in Relation zu der vor dem Reinigungsvorgang aufgebrachten Verunreinigungsmenge),
- Art der noch vorhandenen Verunreinigungen,
- Temperatur- und/oder Druckdaten, die während des Reinigungsvorgangs innerhalb der Reinigungskammer aufgezeichnet wurden, wobei die Messung der jeweiligen Kenngrößen bei Bedarf an unterschiedlichen Stellen innerhalb der Reinigungskammer erfolgen kann (die Daten können ebenfalls als Absolutdaten oder in Relation zu vordefinierten Bezugsgrößen berücksichtigt werden), oder
- Temperaturdaten (insbesondere deren zeitlicher Verlauf während des Reinigungsvorgangs) eines oder mehrerer Prüfkörper, usw.

Insbesondere sieht das Verfahren vor, vor Beginn des Reinigungsvorgangs einen oder mehrere Adapter aus einem Validierungsset auszuwählen, dessen bzw. deren jeweils zweiter Anschlussbereich mit einem der Aufnahmestutzen des Reinigungsapparats koppelbar ist. Der oder die ausgewählten Adapter werden schließlich mit jeweils einem Aufnahmestutzen gekoppelt, wobei zuvor, gleichzeitig oder danach der oder die Aufnahmestutzen mit einem Prüfkörper gekoppelt werden. Der oder die Prüfkörper können somit mit Hilfe der unterschiedlichen Adapter mit den Aufnahmestutzen der meisten verfügbaren Reinigungsgeräte verbunden werden.

Besondere Vorteile bringt es mit sich, wenn vor oder während des Verschließens der Reinigungskammer ein oder mehrere Drucksensoren und/oder ein oder mehrere Temperatursensoren innerhalb der Reinigungskammer platziert werden. Die Sensoren können beispielsweise über das oben beschriebene Halteelement mit dem Deckel oder innerhalb der Reinigungskammer fixiert werden, wobei es von Vorteil ist, wenn die Messfühler der einzelnen Sensoren (wenn mehrere zum Einsatz kommen) die jeweiligen Kenngrößen an unterschiedlichen Stellen innerhalb der Messkammer detektieren. In jedem Fall ist es vorteilhaft, wenn während des Reinigungsvorgangs Druck- und/oder Temperaturdaten von einem oder mehreren der genannten Sensoren detektiert werden, so dass die Bewertung der Reinigungsleistung unter Berücksichtigung der detektierten Druck- und/oder Temperaturdaten erfolgen kann.

Ferner ist es vorteilhaft, wenn der beziehungsweise zumindest einer der Prüfkörper einen den jeweiligen Prüfkörper in Richtung dessen Längsachse durchziehenden Kanal aufweist. Der Kanal oder ein in den Kanal eingesteckter Schlauch kann vor dem Reinigungsvorgang kontrolliert verschmutzt werden, so dass der Prüfkörper selbst nicht verunreinigt werden muss. Während des Reinigungsvorgangs wird der Schlauch von einem von dem Reinigungsapparat abgegebenen Fluid durchströmt. Nach Beendigung des Reinigungsvorgangs kann die Reinigungsleistung unter Berücksichtigung des Reinigungserfolgs des Schlauchs erfolgen.

An dieser Stelle sei klargestellt, dass das Verfahren mit Hilfe eines Validierungssets durchgeführt werden kann, das neben den genannten Adaptern auch die übrigen bisher oder nachfolgend beschriebenen Merkmale aufweisen kann.

Schließlich umfasst die Erfindung die Verwendung eines Validierungssets gemäß bisheriger und/oder nachfolgender Beschreibung zur Durchführung eines Verfahrens gemäß bisheriger und/oder nachfolgender Beschreibung.

Weitere Vorteile der Erfindung sind in den nachfolgenden Ausführungsbeispielen beschrieben. Es zeigen, jeweils schematisch:
- **Figur 1**: einen Reinigungsapparat zum Reinigen von zahnärztlichen Handgeräten,
- **Figur 2**: den Deckel des in Figur 1 gezeigten Reinigungsapparats,
- **Figur 3**: den in Figur 2 gezeigten Deckel, ausgestattet mit Bestandteilen des erfindungsgemäßen Validierungssets, und
- **Figur 4**: ein Halteelement zur Fixierung von Temperatur- oder Drucksensoren.

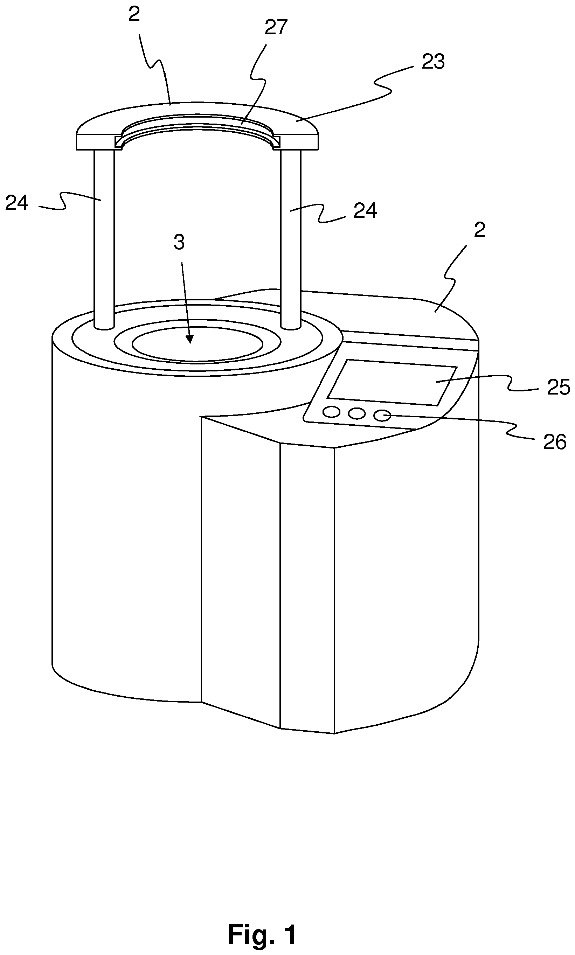

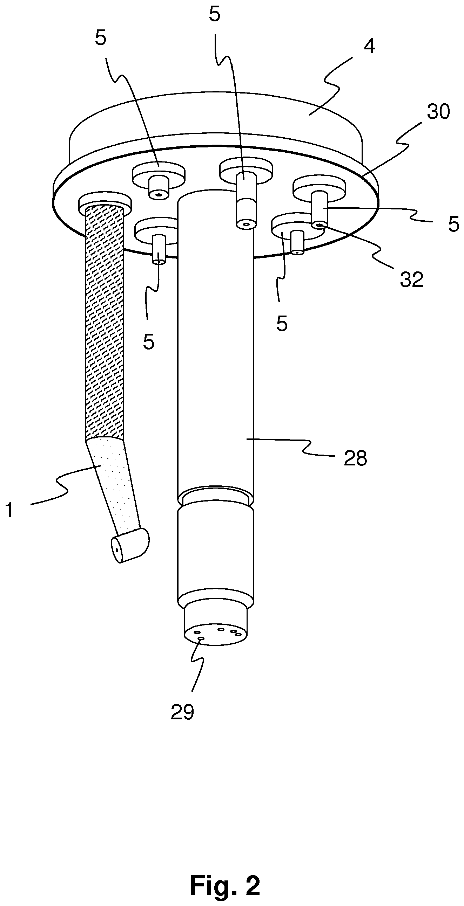

Figur 1 zeigt einen aus dem Stand der Technik bekannten Reinigungsapparat 2 zum Reinigen von zahnärztlichen Handgeräten 1. Ein entsprechendes Handgerät 1 ist in Figur 2 in Form eines Zahnbohrers gezeigt.

Prinzipiell weist der Reinigungsapparat 2 eine Reinigungskammer 3 sowie einen Deckel 4 zum Verschließen der Reinigungskammer 3 auf. Ferner sind in der Regel ein oder mehrere Tasten 26 zum Bedienen des Reinigungsapparats 2 sowie ein Display 25 vorhanden.

Um nun ein oder mehrere zahnärztliche Handgeräte 1 zu reinigen, werden diese bei den meisten bekannten Reinigungsapparaten 2 mit jeweils einem Aufnahmestutzen 5 des Reinigungsapparats 2 gekoppelt, wobei die Aufnahmestutzen 5 beispielsweise, wie in Figur 2 gezeigt, Teil des Deckels 4 sein können.

Im Anschluss daran wird der Deckel 4 auf die Reinigungskammer 3 aufgesetzt oder wie im gezeigten Beispiels in eine Nut 27 eines Deckelhalters 23 geschoben. Der Deckelhalter 23 wird schließlich nach dem Start des Reinigungsvorgangs mit einem Gestänge 24 nach unten verfahren und verschließt letztendlich die Reinigungskammer 3, wobei hier eine Dichtung 30 zum Einsatz kommen kann.

Während des Reinigungsvorgangs werden nun die zahnärztlichen Handgeräte 1 mit einem oder mehreren verschiedenen Fluiden gereinigt, wobei der Eintrag des jeweiligen Fluids ins Innere des Handgeräts 1 über die Aufnahmestutzen 5 erfolgt, die wiederum über ein Verbindungsstück 28 mit einer nicht gezeigten Fluidabgabestelle des Reinigungsapparats 2 in Verbindung stehen. Das Verbindungsstück 28 besitzt hierfür einen oder mehrere innere Fluidkanäle, in die über in Figur 2 sichtbare Fluidöffnungen 29 ein oder mehrere verschiedene Fluide einbringbar sind.

Wie nun Figur 2 beispielhaft zu entnehmen ist, sind verschiedenste Aufnahmestutzen 5 bekannt, die jeweils der Kopplung von zahnärztlichen Handgeräten 1 unterschiedlicher Hersteller dienen.

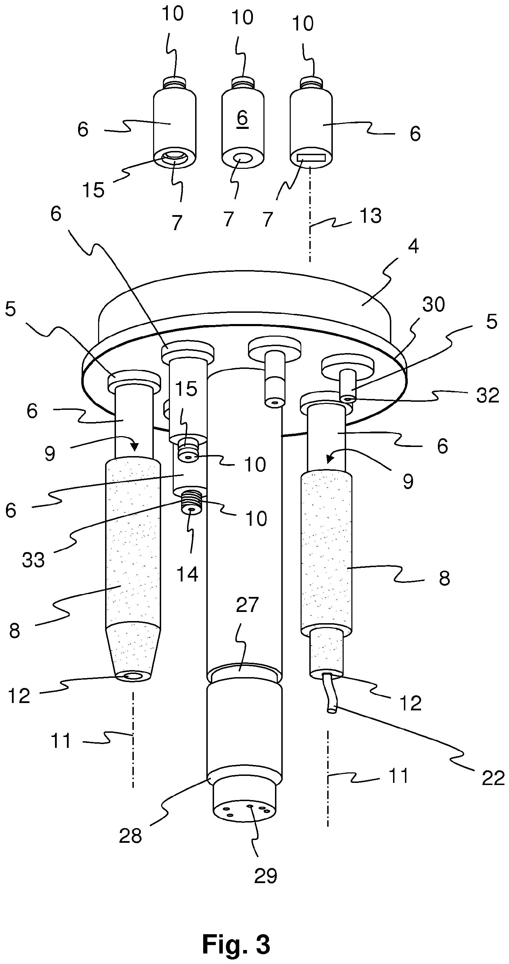

Um nun eine Überprüfung der Reinigungsleistung des Reinigungsapparats 2 durchführen zu können, wird ein Validierungsset vorgeschlagen, wobei der Kern der Erfindung besonders anschaulich aus Figur 3 ersichtlich ist.

Das Validierungsset umfasst einen oder vorzugsweise mehrere Adapter 6. Die Adapter 6 weisen wiederum einen ersten Anschlussbereich 7 und einen zweiten Anschlussbereich 10 auf. Wie aus den drei oben gezeigten Adaptern 6 in Figur 3 ersichtlich, sind die ersten Anschlussbereiche 7 der Adapter 6 vorzugsweise unterschiedlich ausgebildet (wobei selbstverständlich auch mehrere Adaptersätze zum Einsatz kommen können und wobei die Adapter 6 eines Adaptersatzes jeweils gleich ausgebildet sein können). Soll nun eine Validierung des Reinigungsapparats 2 durchgeführt werden, so werden die Adapter 6 aus dem Validierungsset ausgewählt, die mit den Aufnahmestutzen 5 des konkret vorliegenden Reinigungsapparats 2 koppelbar sind.

Die passenden Adapter 6 werden schließlich mit einem Prüfkörper 8 gekoppelt, der über einen Anschluss 9 mit dem zweiten Anschlussbereich 10 je eines Adapters 6 gekoppelt wird (in Figur 3 sind zwei unterschiedlich ausgebildete Prüfkörper 8 dargestellt). Der bzw. die Prüfkörper 8 werden auf diese Weise mit den jeweiligen Aufnahmestutzen 5 verbunden, ohne dass diese für die Validierung geändert oder ausgetauscht werden müssten.

Die Prüfkörper 8 selbst werden vor dem Durchführen des Reinigungsvorgangs entweder kontrolliert verschmutzt oder mit einem oder mehreren Temperatursensoren 17 und/oder Drucksensoren 16 ausgerüstet. Beispielsweise wäre es denkbar, einen entsprechenden Temperatursensor 17 in einen den Prüfkörper 8 in Richtung dessen Längsachse 11 durchziehenden Kanal 12 einzuführen und entsprechend zu fixieren.

Ebenso können ein oder mehrere Prüfkörper 8 mit einem Schlauch 22 (vorzugsweise aus Kunststoff) ausgerüstet werden, wobei sich der Schlauch 22 durch den genannten Kanal 12 des Prüfkörpers 8 und einen entsprechenden Kanal 14 des Adapters 6 erstrecken sollte (wobei sich der Kanal 14 im Wesentlichen entlang einer Längsachse 13 des Adapters 6 erstrecken sollte). Der Schlauch 22 kann vor dem Reinigungsvorgang kontrolliert verschmutzt werden. Durch die Auswertung der Verschmutzung des Schlauchs 22 nach dem Ende des Reinigungsvorgangs sind schließlich Rückschlüsse dahingehend möglich, ob zahnärztliche Handgeräte 1 mit Hilfe des Reinigungsapparats 2 auch im Inneren zuverlässig gereinigt werden (Prüfkörper 8 und Adapter 6 fungieren während des Reinigungsvorgangs also als Dummy eines zahnärztlichen Handgeräts 1).

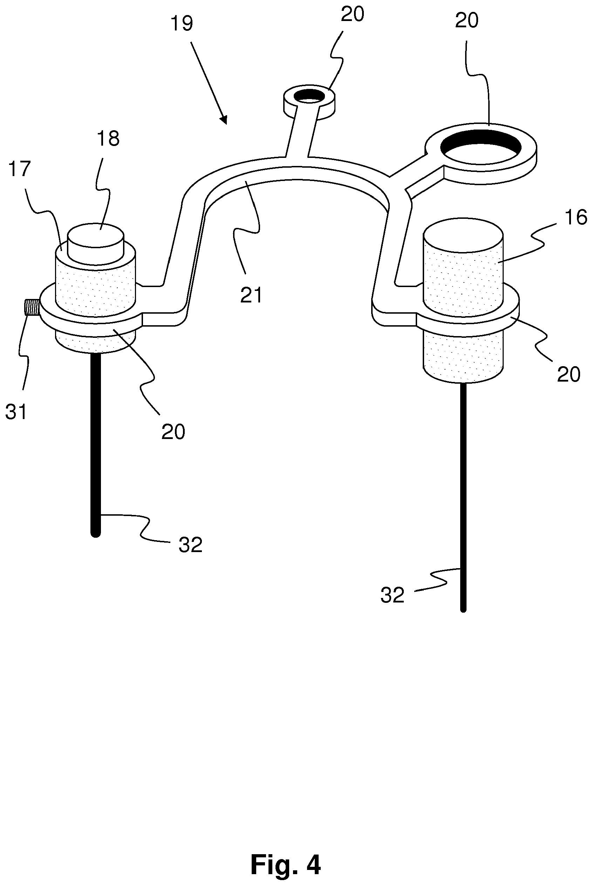

Schließlich zeigt Figur 4 ein mögliches Halteelement in Form einer Halteklammer 19 für mehrere Temperatur- und/oder Drucksensoren 17, 16, wobei das Halteelement ebenfalls Bestandteil des Validierungssets sein kann. In jedem Fall ist es von Vorteil, wenn das Halteelement mehrere Aufnahmen 20 für jeweils einen Temperatursensor 17 oder einen Drucksensor 16 aufweist. Die genannten Sensoren 16, 17 können beispielsweise formschlüssig oder mit Hilfe des in Figur 4 links gezeigten Fixierelements 31 gegenüber der jeweiligen Aufnahme 20 fixiert werden (bei dem Fixierelement 31 kann es sich beispielsweise um eine Schraube oder ein Rastelement handeln).

Das Halteelement kann schließlich über einen Halteabschnitt 21 an das Verbindungsstück 28 des in Figur 3 gezeigten Deckels 4, beispielsweise in einer entsprechenden Nut 27, geklipst werden, so dass die mit dem Haltelement verbundenen Sensoren 16, 17 während des Reinigungsvorgangs ortsfest gehalten werden.

Wie Figur 4 ebenfalls zu entnehmen ist, können die mit Messfühlern 32 ausgestatteten Sensoren 16, 17, die im Übrigen auch Teil des Validierungssets sein können, mit einem Datenspeicher 18 ausgerüstet sein, so dass die während des Reinigungsvorgangs aufgezeichneten Daten zwischengespeichert und nach Beendigung des Reinigungsvorgangs ausgelesen und ausgewertet werden können.

### Bezugszeichenliste

- 1: zahnärztliches Handgerät
- 2: Reinigungsapparat
- 3: Reinigungskammer des Reinigungsapparats
- 4: Deckel des Reinigungsapparats
- 5: Aufnahmestutzen zum Koppeln und Halten eines zahnärztlichen Handgeräts
- 6: Adapter
- 7: erster Anschlussbereich des Adapters
- 8: Prüfkörper
- 9: Anschluss des Prüfkörpers
- 10: zweiter Anschlussbereich des Adapters
- 11: Längsachse des Prüfkörpers
- 12: den Prüfkörper durchziehender Kanal
- 13: Längsachse des Adapters
- 14: den Adapter durchziehenden Kanal
- 15: Rastelement
- 16: Drucksensor
- 17: Temperatursensor
- 18: Datenspeicher
- 19: Halteklammer
- 20: Aufnahme für einen Temperatur- oder Drucksensor
- 21: Halteabschnitt
- 22: Schlauch
- 23: Deckelhalter
- 24: Gestänge
- 25: Display
- 26: Tasten
- 27: Nut
- 28: Verbindungsstück
- 29: Fluidöffnung
- 30: Dichtung
- 31: Fixierelement
- 32: Messfühler

## Patentansprüche

1. Validierungsset zum Überprüfen der Reinigungsleistung eines zum Reinigen von zahnärztlichen Handgeräten (1) ausgelegten Reinigungsapparats (2), wobei der Reinigungsapparat (2) eine Reinigungskammer (3) zur Aufnahme eines oder mehrerer zahnärztlicher Handgeräte (1) sowie einen Deckel (4) zum Verschließen der Reinigungskammer (3) aufweist, und wobei der Reinigungsapparat (2) mehrere Aufnahmestutzen (5) zum Koppeln und Halten eines zahnärztlichen Handgeräts (1) innerhalb der Reinigungskammer (3) aufweist,
dass das Validierungsset einen Adapter (6) umfasst,
der einen ersten Anschlussbereich (7), über den der Adapter (6) mit einem der Aufnahmestutzen (5) des Reinigungsapparats (2) koppelbar ist, und
einen zweiten Anschlussbereich (10) umfasst, wobei das Validierungsset einen Prüfkörper (8) aufweist,
der vor dem Einsetzen in den Reinigungsapparat (2) kontrolliert verschmutzt wird, so dass aus der nach dem Reinigungsvorgang vorhandenen Restschmutzmenge die Reinigungsleistung abgeleitet werden kann, und
der einen Anschluss (9) aufweist, mit dessen Hilfe der Prüfkörper (8) über den zweiten Anschlussbereich (10) des Adapters (6) mit dem Adapter (6) koppelbar ist,
dass das Validierungsset mehrere Adapter (6) umfasst,
**dadurch gekennzeichnet, dass**
zumindest die ersten Anschlussbereiche (7) zweier Adapter (6) unterschiedlich ausgebildet sind, um die jeweiligen Adapter (6) mit unterschiedlich ausgebildeten Aufnahmestutzen (5) koppeln zu können, und
die zweiten Anschlussbereiche (10) aller Adapter (6) gleichartig ausgebildet sind.

2. Validierungsset gemäß dem vorangegangenen Anspruch, **dadurch gekennzeichnet, dass** das Validierungsset mehrere Adaptersätze umfasst, wobei die Adaptersätze jeweils mehrere, vorzugsweise drei, gleichartig ausgebildete Adapter (6) umfassen, und wobei sich die jeweils ersten Anschlussbereiche (7) der Adapter (6) eines Adaptersatzes von den ersten Anschlussbereichen (7) der Adapter (6) des oder der weiteren Adaptersätze unterscheiden.

3. Validierungsset gemäß einem der vorangegangenen Ansprüche, **dadurch gekennzeichnet, dass** der Prüfkörper (8) einen den Prüfkörper (8) in Richtung dessen Längsachse (11) durchziehenden Kanal (12) aufweist, durch den ein vom Reinigungsapparat (2) abgegebenes Fluid strömen kann, wenn der Prüfkörper (8) mit einem der Adapter (6) und der entsprechende Adapter (6) mit einem der Aufnahmestutzen (5) des Reinigungsapparats (2) gekoppelt ist.

4. Validierungsset gemäß einem der vorangegangenen Ansprüche, **dadurch gekennzeichnet, dass** der oder die Adapter (6) jeweils einen den entsprechenden Adapter (6) in Richtung dessen Längsachse (13) durchziehenden Kanal (14) aufweisen, durch den ein vom Reinigungsapparat (2) abgegebenes Fluid strömen kann, wenn der Adapter (6) mit einem Aufnahmestutzen (5) des Reinigungsapparats (2) gekoppelt ist.

5. Validierungsset gemäß einem der vorangegangenen Ansprüche, **dadurch gekennzeichnet, dass** der erste Anschlussbereich (7) der jeweiligen Adapter (6) zumindest ein Rastelement (15) umfasst, mit dessen Hilfe er mit einem Aufnahmestutzen (5) des Reinigungsapparats (2) durch Bildung einer Schnappverbindung koppelbar ist.

6. Validierungsset gemäß einem der vorangegangenen Ansprüche, **dadurch gekennzeichnet, dass** das Validierungsset zumindest zwei unterschiedlich ausgebildete Prüfkörper (8) umfasst, mit deren Hilfe während einer Überprüfung der Reinigungsleistung des Reinigungsapparats (2) unterschiedliche Kenngrößen detektierbar sind.

7. Validierungsset gemäß dem vorangegangenen Anspruch, **dadurch gekennzeichnet, dass** die Anschlüsse der einzelnen Prüfkörper (8), über die sie mit dem genannten zweiten Anschlussbereich (10) eines Adapters (6) koppelbar sind, identisch ausgebildet sind.

8. Validierungsset gemäß einem der vorangegangenen Ansprüche, **dadurch gekennzeichnet, dass** das Validierungsset einen oder mehrere Temperatursensoren (17) und/oder einen oder mehrere Drucksensoren (16) umfasst, wobei der bzw. die Temperatursensoren (17) und/oder der bzw. die Drucksensoren (16) vorzugsweise eine Datenspeicher (18) aufweisen, der ausgebildet ist, vom jeweiligen Sensor gelieferte Messwerte für eine spätere Auswertung zu speichern.

9. Validierungsset gemäß einem der vorangegangenen Ansprüche, **dadurch gekennzeichnet, dass** das Validierungsset wenigstens ein Halteelement, vorzugsweise in Form einer Halteklammer (19), umfasst, wobei das Halteelement Aufnahmen (20) für Temperatursensoren (17) und/oder Drucksensoren (16) aufweist, und wobei das Halteelement einen Halteabschnitt (21) aufweist, über den es mit dem Reinigungsapparat (2) verbindbar ist.

10. Verfahren zum Überprüfen der Reinigungsleistung eines zum Reinigen von zahnärztlichen Handgeräten (1) ausgelegten Reinigungsapparats (2) unter Verwendungs eines Validierungssets nach Ansprüchen 1-9,
- wobei der Reinigungsapparat (2) eine Reinigungskammer (3) zur Aufnahme eines oder mehrerer zahnärztlicher Handgeräte (1) sowie einen Deckel (4) zum Verschließen der Reinigungskammer (3) aufweist, und
- wobei der Reinigungsapparat (2) mehrere Aufnahmestutzen (5) zum Koppeln und Halten eines zahnärztlichen Handgeräts (1) innerhalb der Reinigungskammer (3) aufweist,
**dadurch gekennzeichnet,**
- **dass** vor der Überprüfung der Reinigungsleistung zumindest einer der Aufnahmestutzen (5) mit einem Adapter (6) und der Adapter (6) mit einem Prüfkörper (8) gekoppelt wird,
- **dass** die Reinigungskammer (3) mit Hilfe des Deckels (4) verschlossen wird,
- **dass** anschließend ein Reinigungsvorgang des Reinigungsapparats (2) durchgeführt wird, und
- **dass** der Reinigungsvorgang nach dessen Beendigung bewertet wird, wobei die Bewertung unter Berücksichtigung von während des Reinigungsvorgangs aufgezeichneter physikalischer Daten und/oder unter Berücksichtigung des Reinigungserfolgs des vor Durchführung des Reinigungsvorgangs kontrolliert verschmutzten Prüfkörpers (8) erfolgt.

11. Verfahren gemäß dem vorangegangenen Anspruch, **dadurch gekennzeichnet, dass** vor oder während des Verschließens der Reinigungskammer (3) ein oder mehrere Drucksensoren (16) und/oder ein oder mehrere Temperatursensoren (17) innerhalb der Reinigungskammer (3) platziert werden, dass während des Reinigungsvorgangs Druck- und/oder Temperaturdaten von einem oder mehreren der genannten Sensoren detektiert werden, und dass die Bewertung der Reinigungsleistung unter Berücksichtigung der detektierten Druck- und/oder Temperaturdaten erfolgt.

12. Verfahren gemäß Anspruch 10 oder 11, **dadurch gekennzeichnet, dass** der bzw. zumindest einer der Prüfkörper (8) einen den jeweiligen Prüfkörper (8) in Richtung dessen Längsachse (11) durchziehenden Kanal (12) aufweist, und dass vor dem Verschließen der Reinigungskammer (3) innerhalb des Kanals (12) des bzw. zumindest eines der Prüfkörper (8) ein kontrolliert verschmutzter Schlauch (22) platziert wird, wobei der Schlauch (22) während des Reinigungsvorgangs von einem von dem Reinigungsapparat (2) abgegebenen Fluid durchströmt wird, wobei die Bewertung der Reinigungsleistung unter Berücksichtigung des Reinigungserfolgs des Schlauchs (22) erfolgt.

13. Verwendung eines Validierungssets gemäß einem der Ansprüche 1 bis 9 zur Durchführung eines Verfahrens gemäß einem der Ansprüche 10 bis 12.

## Claims

1. A validation set for checking the cleaning performance of a cleaning apparatus (2) designed for cleaning dental hand tools (1), wherein the cleaning apparatus (2) comprises a cleaning chamber (3) for receiving one or more dental hand tools (1) and a cover (4) for closing the cleaning chamber (3), and wherein the cleaning apparatus (2) has a plurality of receiving sockets (5) for coupling and retaining a dental hand tool (1) within the cleaning chamber (3), that the validation set comprises an adapter (6) having a first connection region (7), via which the adapter (6) can be coupled with one of the receiving sockets (5) of the cleaning apparatus (2), and
comprises a second connection region (10),
wherein the validation set comprises a test piece (8), which is contaminated in a controlled manner prior to insertion into the cleaning apparatus (2), so that the cleaning performance can be derived from the residual amount of contamination present after the cleaning process, and
comprises a connection (9) with the aid of which the test piece (8) can be coupled with the adapter (6) via the second connection region (10) of the adapter (6),
that the validation set comprises a plurality of adapters (6),
**characterized in that**
at least the first connection regions (7) of two adapters (6) are formed differently in order to be able to couple the respective adapters (6) with differently formed receiving sockets (5), and
the second connection regions (10) of all adapters (6) are formed in an identical manner.

2. The validation set according to the preceding claim, **characterized in that** the validation set comprises a plurality of adapter sets, wherein the adapter sets each comprise a plurality, preferably three, of identically formed adapters (6), and wherein the respective first connection regions (7) of the adapters (6) of an adapter set differ from the first connection regions (7) of the adapters (6) or the further adapter set(s).

3. The validation set according to one of the preceding claims, **characterized in that** the test piece (8) comprises a channel (12) passing through the test piece (8) in the direction of its longitudinal axis (11), through which channel a fluid discharged from the cleaning apparatus (2) can flow when the test piece (8) is coupled with one of the adapters (6) and the corresponding adapter (6) is coupled with one of the receiving sockets (5) of the cleaning apparatus (2).

4. The validation set according to one of the preceding claims, **characterized in that** the one or more adapters (6) each comprise a channel (14) passing through the corresponding adapter (6) in the direction of its longitudinal axis (13), through which a fluid discharged from the cleaning apparatus (2) can flow when the adapter (6) is coupled with a receiving socket (5) of the cleaning apparatus (2).

5. The validation set according to one of the preceding claims, **characterized in that** the first connection region (7) of the respective adapters (6) comprises at least one latching element (15) with the aid of which it can be coupled with a receiving socket (5) of the cleaning apparatus (2) by forming a snap-action connection.

6. The validation set according to one of the preceding claims, **characterized in that** the validation set comprises at least two differently formed test pieces (8), with the aid of which different characteristic parameters can be detected during a check of the cleaning performance of the cleaning apparatus (2).

7. The validation set according to the preceding claim, **characterized in that** the connections of the individual test pieces (8) are formed identically, via which connections they can be coupled with said second connection region (10) of an adapter (6).

8. The validation set according to one of the preceding claims, **characterized in that** the validation set comprises one or more temperature sensors (17) and/or one or more pressure sensors (16), wherein the temperature sensor(s) (17) and/or the pressure sensor(s) (16) preferably comprise a data memory (18) which is formed to store measured values supplied by the respective sensor for later evaluation.

9. The validation set according to one of the preceding claims, **characterized in that** the validation set comprises at least one retaining element, preferably in the form of a retaining clip (19), wherein the retaining element comprises receptacles (20) for temperature sensors (17) and/or pressure sensors (16), and wherein the retaining element comprises a retaining section (21), via which it is connectable to the cleaning apparatus (2).

10. A method for checking the cleaning performance of a cleaning apparatus (2) designed for cleaning dental hand tools (1) using a validation set according to claims 1-9,
- wherein the cleaning apparatus (2) comprises a cleaning chamber (3) for receiving one or more dental hand tools (1) and a cover (4) for closing the cleaning chamber (3), and
- wherein the cleaning apparatus (2) comprises a plurality of receiving sockets (5) for coupling and retaining a dental hand tool (1) within the cleaning chamber (3),
**characterized in that**
- before checking the cleaning performance, at least one of the receiving sockets (5) is coupled with an adapter (6) and the adapter (6) is coupled with a test piece (8),
- that the cleaning chamber (3) is closed with the aid of the cover (4),
- that a cleaning process of the cleaning apparatus (2) is then performed, and
- that the cleaning process is evaluated after its completion, wherein the evaluation is performed taking into account physical data recorded during the cleaning process and/or taking into account the cleaning outcome of the test piece (8) which has been contaminated in a controlled manner before carrying out the cleaning process.

11. The method according to the preceding claim, **characterized in that** one or more pressure sensors (16) and/or one or more temperature sensors (17) are placed within the cleaning chamber (3) before or during the closure of the cleaning chamber (3), that during the cleaning process, pressure and/or temperature data of one or more said sensors are detected, and that the evaluation of the cleaning performance is performed taking into account the detected pressure and/or temperature data.

12. The method according to claim 10 or 11, **characterized in that** the or at least one of the test pieces (8) comprises a channel (12) passing through the respective test piece (8) in the direction of its longitudinal axis (11), and **in that** before closing the cleaning chamber (3), a hose (22) contaminated in a controlled manner is placed within the channel (12) of the or at least one of the test pieces (8), wherein a fluid discharged from the cleaning apparatus (2) passes though the hose (22) during the cleaning process, wherein the evaluation of the cleaning performance is performed taking into account the cleaning outcome of the hose (22).

13. The use of a validation set according to one of claims 1 to 9 for performing a method according to one of claims 10 to 12.

## Revendications

1. Kit de validation pour vérifier la performance de nettoyage d'un appareil de nettoyage (2) conçu pour nettoyer des appareils de médecine dentaire portatifs (1), sachant que l'appareil de nettoyage (2) comprend une chambre de nettoyage (3) pour recevoir un ou plusieurs appareils de médecine dentaire (1) ainsi qu'un couvercle (4) pour fermer la chambre de nettoyage (3), et sachant que l'appareil de nettoyage (2) comportant une multitude de tubulures de réception (5) pour coupler et tenir un appareil de médecine dentaire portatif (1) au sein de la chambre de nettoyage (3), le kit de validation comprend un adaptateur (6) qui comporte une première zone de connexion (7) par laquelle l'adaptateur (6) peut être couplé à l'une des tubulures de réception (5) de l'appareil de nettoyage (2), et comporte une seconde zone de connexion (10),
sachant que le kit de validation comprend un corps d'essai (8) qui est contaminé d'une manière contrôlée avant d'être mis en place dans l'appareil de nettoyage (2), de sorte que la performance de nettoyage puisse être dérivée de la quantité de saleté résiduelle présente après l'opération de nettoyage, et
qui présente un raccordement (9) à l'aide duquel le corps d'essai (8) peut être couplé à l'adaptateur (6) via la seconde zone de connexion (10) de l'adaptateur (6),
le kit de validation comprend plusieurs adaptateurs (6),
**caractérisé en ce que**
au moins les premières zones de connexion (7) de deux adaptateurs (6) se présentent sous des formes différentes afin de pouvoir coupler les adaptateurs (6) respectifs à des tubulures de réception (5) de formes différentes, et
les secondes zones de connexion (10) de tous les adaptateurs (6) se présentent sous des formes de même type.

2. Kit de validation selon la revendication précédente, **caractérisé en ce que** le kit de validation comprend une multitude de jeux d'adaptateurs, sachant que les jeux d'adaptateurs comprennent respectivement une multitude d'adaptateurs (6), de préférence trois, se présentant sous une forme de même type, et sachant que les premières zones de connexion (7) respectives des adaptateurs (6) d'un jeu d'adaptateurs diffèrent des premières zones de connexion (7) des adaptateurs (6) du ou des jeux d'adaptateurs supplémentaires.

3. Kit de validation selon l'une des revendications précédentes, **caractérisé en ce que** le corps d'essai (8) présente un canal (12) qui traverse le corps d'essai (8) dans la direction de son axe longitudinal (11), à travers lequel peut circuler un fluide déchargé par l'appareil de nettoyage (2) lorsque le corps d'essai (8) est couplé à l'un des adaptateurs (6) et l'adaptateur (6) afférent est couplé à l'une des tubulures de réception (5) de l'appareil de nettoyage (2).

4. Kit de validation selon l'une des revendications précédentes, **caractérisé en ce que** le corps d'essai (6) présente un canal (14) qui traverse le corps d'essai (6) dans la direction de son axe longitudinal (13), à travers lequel peut circuler un fluide déchargé par l'appareil de nettoyage (2) lorsque le corps d'essai (8) est couplé à l'un des adaptateurs (6) et l'adaptateur (6) afférent est couplé à l'une des tubulures de réception (5) de l'appareil de nettoyage (2).

5. Kit de validation selon l'une des revendications précédentes, **caractérisé en ce que** la première zone de connexion (7) des adaptateurs (6) respectifs comprend au moins un élément d'encliquetage (15) à l'aide duquel il peut être couplé à une tubulure de réception (5) de l'appareil de nettoyage (2) par formation d'une liaison par encliquetage.

6. Kit de validation selon l'une des revendications précédentes, **caractérisé en ce que** le kit de validation comprend au moins deux corps d'essai (8) de formes différentes, à l'aide desquels des caractéristiques de performance différentes peuvent être détectées lors d'une vérification de la performance de nettoyage de l'appareil de nettoyage (2).

7. Kit de validation selon la revendication précédente, **caractérisé en ce que** les raccordements des corps d'essai (8) individuels, par lesquels ils peuvent être couplés à ladite seconde zone de connexion (10) d'un adaptateur (6), sont de forme identique.

8. Kit de validation selon l'une des revendications précédentes, **caractérisé en ce que** le kit de validation comprend un ou plusieurs capteurs de température (17) et/ou un ou plusieurs capteurs de pression (16), sachant que le ou les capteur(s) de température (17) et/ou le ou les capteur(s) de pression (16) comporte(nt) de préférence une mémoire de données (18) qui se présente sous une forme pour enregistrer des valeurs de mesure délivrées par le capteur respectif en vue d'une évaluation ultérieure.

9. Kit de validation selon l'une des revendications précédentes, **caractérisé en ce que** le kit de validation comprend au moins un élément d'arrêt, de préférence sous la forme d'une agrafe de retenue (19), sachant que l'élément d'arrêt présente des logements (20) pour les capteurs de température (17) et/ou les capteurs de pression (16), et sachant que l'élément d'arrêt présente une section de retenue (21) par laquelle il peut être relié à l'appareil de nettoyage (2).

10. Procédé pour vérifier la performance de nettoyage d'un appareil de nettoyage (2) conçu pour nettoyer des appareils de médecine dentaire portatifs (1) par utilisation d'un kit de validation selon les revendications 1-9,
- sachant que l'appareil de nettoyage (2) comprend une chambre de nettoyage (3) pour recevoir un ou plusieurs appareils de médecine dentaire portatifs (1) et un couvercle (4) pour fermer la chambre de nettoyage (3), et
- sachant que l'appareil de nettoyage (2) comporte une multitude de tubulures de réception (5) pour coupler et tenir un appareil de médecine dentaire portatif (1) au sein de la chambre de nettoyage (3), **caractérisé en ce que**,
- avant la vérification de la performance de nettoyage, au moins l'une des tubulures de réception (5) est couplée à un adaptateur (6) et l'adaptateur (6) est couplé à un corps d'essai (8),
- que la chambre de nettoyage (3) est fermée à l'aide du couvercle (4),
- qu'une opération de nettoyage de l'appareil de nettoyage (2) est effectuée, et
- que l'opération de nettoyage est évaluée après son achèvement, sachant que l'évaluation est effectuée compte tenu de données physiques enregistrées au cours de l'opération de nettoyage et/ou compte tenu du succès du nettoyage du corps d'essai (8) qui a été contaminé de manière contrôlée avant l'exécution de l'opération de nettoyage.

11. Procédé selon la revendication précédente, **caractérisé en ce que**, avant ou pendant la fermeture de la chambre de nettoyage (3), un ou plusieurs capteur(s) de pression (16) et/ou un ou plusieurs capteur(s) de température (17) sont placés à l'intérieur de la chambre de nettoyage (3), **en ce que** des données de pression et/ou de température d'un ou plusieurs des capteurs cités sont détectées pendant l'opération de nettoyage et **en ce que** l'évaluation de la performance de nettoyage est effectuée compte tenu des données de pression et/ou de température détectées.

12. Procédé selon la revendication 10 ou 11, **caractérisé en ce que** le ou au moins l'un des corps d'essai (8) présente un canal (12) traversant le corps d'essai (8) respectif dans la direction de son axe longitudinal (11), et **en ce que**, avant la fermeture de la chambre de nettoyage (3), un tuyau (22) souillé de manière contrôlée est placé dans le canal (12) du ou au moins de l'un des corps d'essai (8), sachant que le tuyau (22) est parcouru par un fluide délivré par l'appareil de nettoyage (2) pendant l'opération de nettoyage, sachant que l'évaluation de la performance de nettoyage est effectuée compte tenu du succès du nettoyage du tuyau (22).

13. Utilisation d'un kit de validation selon l'une des revendications 1 à 9 pour exécuter un procédé selon l'une des revendications 10 à 12.
